# EUROPEAN PATENT APPLICATION

(11) **EP 1 930 074 A1**
(43) Date of publication of application: **11.06.2008**
(21) Application number: 06025439.8
(22) Date of filing: 08.12.2006
(51) Int. Cl.: B01J 23/28, B01J 27/057, B01J 29/03, C07C 51/215, B01J 35/10, B01J 37/03

(54) **Novel mesoporous mixed metal oxide catalyst and method for the preparation thereof**

(71) Applicant: Schlögl, Robert, Prof. Dr., 12161 Berlin (DE); Trunschke, Annette, Dr., 12489 Berlin (DE)
(72) Inventor: Schlögl, Robert, Prof. Dr., 12161 Berlin (DE); Trunschke, Annette, Dr., 12489 Berlin (DE)
(74) Representative: HOFFMANN EITLE

(57) **Abstract**

The present invention relates to novel mesoporous mixed metal oxide catalysts and a method for the preparation thereof, as well as the use thereof as a catalyst in the oxidation of hydrocarbons or partially oxidized hydrocarbons. More specifically, the present invention relates to mesoporous mixed metal oxide catalysts comprising at least two, preferably at least three different metal species, at least one of which belongs to the group of transition metals, a method for the preparation thereof, which comprises a production step over the neutral templating route and a calcination step in a substantially oxygen-free atmosphere at a temperature between 300 to 700°C, and the use of such catalysts as oxidation catalyst in the preparation of oxidized hydrocarbons, and especially the selective oxidation or ammoxidation of propane to acrylic acid and acrylonitrile, respectively. A more preferred catalyst comprises Mo, V, Te and Nb.

## Description

### FIELD OF THE INVENTION

The present invention relates to novel mesoporous mixed metal oxide catalysts and a method for the preparation thereof, as well as the use thereof as a catalyst in the oxidation of hydrocarbons or partially oxidized hydrocarbons. More specifically, the present invention relates to mesoporous mixed metal oxide catalysts comprising at least two, preferably at least three different metal species, at least one of which belongs to the group of transition metals, a method for the preparation thereof, which comprises a production step over the neutral templating route, and the use of such catalysts as oxidation catalyst in the preparation of oxidized hydrocarbons, and especially the selective oxidation or ammoxidation of propane to acrylic acid and acrylonitrile, respectively. A more preferred catalyst comprises Mo, V, Te and Nb.

### BACKGROUND ART

Bulk and supported mixed metal oxide catalysts are an important class of catalytic materials, which are employed in numerous industrial processes. They are used as oxidation catalysts in many reactions, including the preparation of various basic chemicals. Among them, unsaturated aldehydes and carboxylic acids, such as (meth)acrylic acid and esters thereof, are important starting materials for the production of a broad spectrum of oligomeric and polymeric products.

The production of unsaturated carboxylic acids by oxidation of an olefin or an alkane is well known in the art. For example, acrylic acid may be prepared by oxidizing propane or propylene in the gas phase. Similarly, methacrylic acid can be prepared by gas phase oxidation of isobutene or isobutane. Alternatively, the oxidation could also be conducted using already partially oxidized intermediates as starting materials, such as acrolein or methacrolein.

Metal oxide catalysts used for the above types of reactions are manifold and are well known to the person skilled in the art. However, despite the fact that many different and suitable catalyst for the present type of catalytic oxidation are known, the conversion rate and/or the selectivity towards the desired product is not always satisfactory. As a result, the product yield (productivity) is oftentimes too low. Thus, continuous efforts are undertaken by many researchers to obtain catalysts showing an improved conversion rate and/or selectivity, and the provision of better catalysts is an ongoing challenge.

Among the known metal oxide catalysts, also catalysts containing oxides of molybdenum, vanadium and tellurium (Mo-V-Te catalysts) are well known in the state of the art. Catalysts wherein the above metal oxides are supplemented with niobium oxide and optionally further metal oxide components are described in e.g. US 5,380,933. Such catalysts also have been subject to scientific studies concerning the oxidative dehydrogenation of hydrocarbons, e.g. propane, as well as the selective oxidation to the respective acrylic acids, see Zhen Zhao et al., J.Phys.Chem. B 2003, 107, 6333-6342, and D. Vitry et al., Applied Catalysis A: General 251 (2003) 411-424.

The production of (meth)acrylic acid by a gas phase catalytic oxidation of a mixtures of propane/propene, or isobutene/isobutane is disclosed in US 6,710,207.

The catalysts discussed above are bulk materials. However, due to their bigger surface area (and consequently the possibility of increased conversion rates per mass of catalysts), the use of porous catalysts having a high surface area is highly desired. Additionally, the use of porous materials as catalysts can lead to improved selectivity as compared to the respective bulk materials due to effects such as site isolation, which may open an alternative reaction pathway.

Porous solids can broadly be classified into microporous (with pore diameters of 2 nm or less) and mesoporous materials (with pore diameters of 2 - 50 nm). Due to the size of the organic substrate, mesoporous materials are more promising as catalysts.

The application potential of well ordered mesoporous silica in catalysis has been recognized in the early nineties of the previous century (see, for example, C. T. Kresge et al. in Nature, Vol. 359, October 22, 1992). Such mesoporous solids (having pore diameters of 2 to 50 nm) are typically synthesized via a so-called "templating route", wherein the precursor(s) of the mesoporous material is polymerized (e.g., condensed) around a templating agent, such as micelles in aqueous solution. Examples of such materials include SBA-15, MCM-41, MCM-48, ZSM-5 or silicalite-1. These porous materials can be broadly classified into those prepared by a nonionic (neutral) templating route (using, for example, dodecyl amine, as in the case of HMS, or block polymers, such as triblock copolymers of the ethylenoxide/propylenoxide/ethylenoxide type, or diblock copolymers of the ethylenoxide/butylenoxide-type, such as in the preparation of SBA-15), and those using an ionic (charged) template (such as hexadecyltrimethylammonium bromide, as in the case of MCM-41). Whilst the focus of most of the research undertaken in this field was on siliceous and/or alumina-derived materials, the synthesis strategies applied in the preparation of such materials have recently also been extended to non-siliceous materials. These synthesis strategies and the materials derived therefrom are covered by a large number of reviews, for example in G.J.A.A. Soler-Illia, et al., Chem. Rev. 2002, 102, 4093-4138; F. Schüth, Chem. Mater. 2001, 13, 3184-3195, B. Smarsly, M. Antonietti, Eur. J. Inorg. Chem. 2006, 1111, and F. Schüth and A. Taguchi in "Microporous and Mesoporous materials" 77, 2005, 1-45.

The diversity of transition metal chemistry results in various synthetic approaches using different templating agents, such as ionic (charged) and neutral surfactants, triblock copolymers or ligand-assisted templating routes. The complications originating from the high redox activity of transition metal oxides often lead to a collapse of the mesophase during template removal by calcinations. If non-ionic surfactants are used, the weak interaction between the inorganic species and the template, such as hydrogen bonding, creates the mesophase. However, despite the numerous efforts in this respect, the preparation of mesoporous mixed metal oxides has so far been a difficult task, and oftentimes the materials prepared via such a route show a collapsed structure and/or no mesoporosity.

X. He, M. Trudeau, and D. Antonelli describe in Inorg. Chem. 2001, 40, 6463 - 6468 the synthesis and electronic properties of Low-Dimensional Bis(benzene)vanadium reduced mesoporous niobium oxide composites. The preparation method described in this publication utilizes commercial mesoporous niobium oxide (Nb-TMS1), obtainable from Aldrich, which is subsequently treated with bis(benzene)vanadium. The preparation method does not involve a calcination step in a substantially oxygen-free atmosphere at a temperature between 300 to 700°C. Instead, the treated Nb-TMS1 is dried in vacuo using a Schlenk line. In an alternative approach described in this document, the as-synthesized bis(benzene)vanadium composites are oxidized in air at 100 °C for 24 hours, followed by reduction with K-naphtalene. The resulting material is insulating.

L. Yuan, S. Bhatt, G. Beaucage, V. V. Guliants, S. Mamedov and R. S. Soman describe in J. Phys. Chem. B 2005, 109, 23250-23254 mesoporous mixed Nb-M(M=V, Mo, and Sb)oxides for the oxidative dehydrogenation of propane. The synthesis method of the binary oxides described in this document involves a calcination treatment in air at 400 - 600 °C for 3 - 5 hours in order to remove the surfactant (templating agent).

A number of thermally stable binary and mixed transition metal oxides, such as titania, zirconia, niobia, tungsten oxide or zirconia-titania, were prepared from metal chloride precursors in the presence of non-ionic triblock copolymers containing poly-(ethylenoxide)ₓ,-poly-(propylenoxide)_{y}-poly(ethylenoxide)ₓ (Pluronic P-123) in ethanolic solution. The mesostructures of vandadium and molybdenum oxide by the same method collapsed during template removal by calcination at 573 to 873 K in air. However, the preparation of thermally stable mesoporous vanadium oxide have failed so far, whereas thermally stable niobium-vanadium oxide has been reported.

Complex mixed oxides (specifically those containing Mo, V, Te, and optionally Nb) are particularly promising as catalysts for the selective oxidation or ammoxidation of propane to acrylic acid and acrylonitrile, respectively. The activated catalysts are generally composed of essentially two orthorhombic phases, designated as M1 and M2, respectively. These crystalline materials usually show surface areas below 20 m² g⁻¹. Recently, Florea et al. repoted enhanced surface areas of MoVTeNb oxide catalysts by adding citric acid and/or cetyl trimethyl ammonium bromide (CTAB) during the preparation (see M. Florea et al., Catalysis today, 2006, 112, 139). However, in order to remove the organic additives, the calcination step was performed in air at 773 K, resulting in complex mixtures of crystalline binary and mixed oxides, indicating that the mesostructure has at least partially collapsed.

As described above, many attempts to prepare mesoporous mixed metal oxide catalysts have been made. However, the preparation of stable mesoporous materials remains a difficult task. Additionally, it is noted that the electronic properties of mesoporous mixed metal oxides (i.e. the oxidation state of the metals comprised therein) often differ from those of the corresponding bulk (non-porous) materials. Specifically, the preparation of mesoporous mixed metal oxides via a templating route oftentimes leads to materials having significantly different electronic properties, i.e. having electronic properties different from the corresponding bulk (non-porous) material. In this connection it should be taken into account that the preparation steps performed during the synthesis of mesoporous mixed metal oxides (such as the removal of the templating agent, a calcination treatment in air etc.) are likely to influence the electronic properties of the obtained mesoporous mixed metal oxide. Moreover, the synthesis of mesoporous materials via calcination in air oftentimes leads to materials having at least some crystalline domains, which is undesirable in view of the uniformity of the material and the regularity of the mesoporous structure. Accordingly, no preparation method for the preparation of mesoporous mixed metal oxides having a certain stoichiometric composition with electronic properties identical or highly similar to the corresponding bulk material is known.

### SUMMARY OF THE INVENTION

An object of the present invention is therefore to solve the problems associated with the prior art. Specifically, it is an object of the present invention to provide novel mixed metal oxides having a mesoporous structure, which contain at least one transition metal, and a novel method for the preparation thereof.

It is another object of the present invention to provide novel mesoporous mixed metal oxide catalysts having higher surface areas as compared to the bulk (non-porous) material (preferably at least 20, more preferably at least 50, even more preferably more than 70, and most preferably more than 100 m²/g), and a method for the production thereof.

A still further object of the present invention is to provide a method for the preparation of mesoporous mixed metal oxides via a templating route that does not significantly affect the electronic properties of the metal species contained therein as compared to the bulk (non-porous) material obtained from the same metal precursors in the same stoichiometric composition. Simultaneously, such preparation method shall not exclude that specific electronic properties of the mixed metal oxide can be tuned with regard to specific applications of the mixed metal oxide, during or after its synthesis.

The catalysts (i.e. the mixed metal oxides) of the invention comprise at least two, preferably at least three different metal species, at least one of which is a transition metal, which is preferably molybdenum. More preferably, the catalyst comprises at least two transition metals selected from Mo, V and Nb, in particular Mo and V. Even more preferably, the catalyst comprises all of these three elements, and it is still more preferred that the catalyst comprises these three elements (V, Nb and Mo), and additionally Te.

More preferred catalysts of the invention form a composition according to the formula (I) shown below:

MoVₐTe_{b}Nb_{c}Z_{d}Oₓ (I)

wherein a = 0.0-0.50, preferably 0.15 - 0.40, b = 0-0.45, preferably 0.10-0.40, c = 0 - 0.5, preferably 0.07 - 0.25, d ≤ 0.05 and x is a number depending on the relative amount and valence of the elements different from Oxygen in formula (I), and Z is at least one element selected from Ru, Mn, Sc, Ti, Cr, Fe, Co, Ni, Cu, Zn, Ga, Y, Zr, Rh, Pd, In, Sb, Ce, Pr, Nd, Te, Sm, Tb, Ta, W, Re, Ir, Pt, Au, Pb, and Bi, provided that at least two, more preferably at least three different metal species are contained in the catalyst composition, that is, one, more preferably at least two, of a, b, c, and d are not zero at the same time. "Average" composition means the composition as can be determined with techniques such as XRF suitable for analyzing the bulk elemental composition.

Preferably, in formula (I) a = 0,15-0,40, b = 0,15-0,40, c = 0,07-0,25, and d = 0,03 or less, more preferably a = 0,15-0,30, b = 0,15-0,25, c = 0,10-0,23 and d = 0,01 or less.

According to one preferred embodiment of the present invention d is 0 in formula (I).

If in the compound of the formula (I) the at least one optional element Z is present (i.e. d > 0), it is preferably at least one element selected from Ru, Mn, Cr, Fe, Co, Ni, Zr, Rh, Pd, In, Sb, Ce, Ta, W, Pt, and Bi. More preferred are compounds of formula (I), wherein Z, if present, is at least one element selected from Cr and Ni. Another preferred embodiment relates to the use of Ru, Cu, Rh, Re and/or Mn as Z element, Ru, Mn and Cu, in particular Ru and Mn being particularly preferred. If element Z is present, the lower limit of d is preferably 0,0005, in particular 0,001.

More preferred catalysts have the stoichiometric composition Mo₁V_{0.19-0.25}Te_{0.19-0.23}Nb_{0.12-0.20}Oₓ, wherein x is defined as in formula (I) above

Another object of the present invention is to provide a method for the production of the catalysts described above, in particular those according to formula (I) shown above. The use of such catalysts and catalysts derived from the method of the present application is also within the scope of this invention.

Still another object of the present invention is, in a preferred embodiment, to provide a method for the preparation of mesoporous mixed metal oxides according to formula (I) above, which are substantially free of crystalline domains having long range atomic order (i.e. which show no well-defined diffraction peaks, as defined below), in particular those attributable to Mo(VI)species, such as, for example, MoO₃.

### DETAILED DESCRIPTION OF THE INVENTION

### A) Preparation method according to the invention

The preparation method of the invention is a method for preparing mesoporous mixed metal oxide catalysts. The method is characterized as set out in the following items 1) to 4).
1) A method for the preparation of a mesoporous mixed metal oxide catalyst, comprising:
   (i) a step of providing metal precursors and a neutral templating agent in a solution or slurry,
   (ii) a step of reacting the mixture obtained in (i) and
   (iii) a step of removing the template from the material obtained from (ii), comprising a calcinations step in a substantially oxygen-free atmosphere at a temperature between 300 to 700°C.
2) The method according to item 1) above, wherein step ii) is performed in a pressure-resistant vessel
3) The method according to any of items 1) or 2) above, wherein step (ii) is performed at a temperature between 100°C and 200°C.
4) The method according to anyone of items 1) to 3) above, wherein the template is at least partially removed from the material obtained from step (ii) prior to the calcination treatment in step (iii).

Further embodiments of the method of the present invention will become apparent from the following detailed description.

### STEP (i)

### Metal precursors

The metal precursors (metal sources) used in step (i) of the method of the invention are not particularly limited, as long as they can provide the respective metals contained in the final metal oxide catalyst. Preferably, the metal precursors do not contain counter-ions that are present in the final catalysts after the calcination step (iii). Suitable metal precursors that do not contain such counter-anions are the respective acids, hydroxides or oxides. Furthermore, salts of the metal precursors that contain counter-ions which are removable by a calcination treatment according to step (iii) can also preferably be used, which include the respective organic counter-ions, such as the respective oxalates, acetates, or acetylacetonates. Ammonium salts can also preferably be used. Further metal precursors usable in the method of the present invention include the respective sulphates, nitrates, and halogenides. Heteropolyacids and their salts, with e.g. Anderson or Keggin structure, can also be used as precursors.

Suitable starting materials for Mo, V, Te and Nb oxides are for instance those described in US 5,380,933 (col. 3, line 27 to 57) and/or US 6,710,207 (col. 8, lines 12 to 30), including the preferred ammonium para- or heptamolybdate, ammonium metavanadate, telluric acid and ammonium niobium oxalate. Preferably, a solution of the V source (e.g. an aqueous ammonium metavanadate solution or an aqueous vanadium (IV) oxide sulphate hydrate (VOSO₄ * xH₂O)) and a solution of the Te source (e.g. an aqueous solution of telluric acid) are added to a solution of the Mo source (e.g. an aqueous solution of ammonium heptamolybdate), preferably after heating the Mo solution, followed by the addition of the solution of a Nb source (e.g. an aqueous solution of ammonium niobium oxalate). The V source preferably comprises or consists of a V (IV) species.

Similarly, a suitable starting material for the optional Z element can be selected by a skilled person from those used in the art. Mnaganese (Mn) can for instance be added as manganese acetate and ruthenium (Ru) as polyacid, for instance Mo-containing (optionally also P-containing) polyacids such as H₃PMo₁₁RuO₄₀.
Additionally, in one embodiment the metal precursor can be in a form, in which a group is eliminated during the course of the reaction performed in step (ii) of polymerizable compounds, such as in the form of the metal alkoxides (e.g. methoxide, ethoxide etc.). In another preferable embodiment, alkoxide-free metal species are used as metal precursors.

According to one preferred embodiment of the present invention, the amounts of starting materials are adjusted as precisely as possible since this appears to have a great impact on the activity of the target catalyst. Preferably, the concentration (by mol) of each metal existing in the starting composition should not differ more than 1% from the calculated composition for a given catalyst system. Differences of not more than 0.5%, in particular not more than 0.1% by mol are more preferred. This can be achieved by verifying the actual content of the individual catalyst metal in the solutions used, e.g. by titration control and/or using metering devices for dosing the metal solutions as precisely as possible.

### Solvents

In step (i) of the method of the present invention, the above-described metal precursors are provided in a solution or a slurry.

The type of the solvent used in this regard is not particularly limited, as long as it can dissolve the metal precursors at least to some degree. If a pressure-resistant vessel is used in step (ii) of the method of the present invention, it is sufficient if the solvent used can dissolve the metal precursors at least to some degree under conditions in the pressure-resistant vessel. That is, in this case a solvent can be used which does not dissolve the metal precursors under standard conditions (room temperature and 1 atm pressure), but which can dissolve the metal precursors at least to some degree during the conditions present in step (ii) if a pressure-resistant vessel is used (elevated pressure and/or temperature).

Preferred solvents comprise water and/or one or more polar solvents, such as protic ones, e.g. alcohols (e.g. methanol, ethanol, i-propanol), or aprotic ones, such as ketones (e.g. acetone) or ethers (e.g. dimethylether, diethylether, di-tert.butylether). The most preferred solvent is water. When water or a water-containing solvent is used, the solution or slurry may be denoted as "aqueous" solution or slurry.

Besides the metal precursor, the templating agent and the solvent, the solution or slurry provided in step (i) of the method of the invention can contain various additives. Such additives can be used to tailor the pore size of the resulting catalyst, for example due to incorporation thereof in the templating agent, increasing its size. Other additives are for example agents that complex the metal or that increase its solubility, for instance due to so-called masking. As suitable additives in this regard mention can be made of citric acid, oxalic acid or EDTA (ethylene diamine tetra acetic acid). Furthermore, reducing agents, such as hydrazine or hydroxylamine, can be used to control the oxidation state of the metal in the synthesis. Oxalic acid is a useful additive to adjust the oxidation state of tellurium when the synthesis is performed in a pressure resistant vessel or during thermal treatment. Solids, which are not dissolved under the synthesis conditions in the pressure resistant vessel, can be added as diluents. Examples are particles of oxides like silica, SiC or carbon, such as activated carbon or nanostructured carbon, such as carbon nanotubes or nanofibres..

### Non-ionic (neutral) templating agent

In the method of the present invention, the non-ionic (neutral) templating agent present in the solution or slurry provided in step (i) of the method of the present invention serves as a template for the structure (the pores) of the final catalysts. Hereby the shape and size of the templating agent determines the shape and size of the pores present in the mesoporous catalyst obtained after step (iii) of the method of the present invention.

The preferred templating agents possess a free electron pair, as present, for instance, in compounds having an ether bond, where a free electron pair is present at the respective oxygen atom. These can also be denoted as Lewis-bases.

As discussed above, templating agents used in the preparation of mesoporous solids can broadly be classified into neutral (i.e. those that do not dissociate in ions in water) templating agents and charged templating agents (i.e. those carrying a charge before or after dissociation in water, such as salts, for instance CTAB). As charged templating agents are more difficult to remove from the synthesized solid, and further considering that the structure of porous metal oxides prepared using a charged templating agent oftentimes collapses during or after removal of the template, a neutral templating agent is used in the present invention.

The neutral templating agent used in the method of the present invention is not particularly limited, as long as it does neither carry a positive or negative charge nor dissociates in water in species carrying a positive or negative charge. Preferred templating agents used in the method of the present invention are oxygen-containing copolymers, such as poly(alkylene oxide) polymers, for instance poly(ethylene oxide), triblock copolymers of the poly(alkylene oxide) type, such as those of the ethylenoxide/propylenoxide/ethylenoxide type, or diblock copolymers of the poly(alkylene oxide) type, such as those of the ethylenoxide/butylenoxide-type. These polymers are available under the tradename Pluronic. A particularly preferred templating agent is Pluronic-P123.

As discussed above, the size and shape of the templating agent determines the structure of the resulting mesoporous material. Accordingly, the pore size can be tuned by adjusting the size of the templating agent. In the case of templating agents of the type of oxygen-containing copolymers, such as poly(alkylene oxide) polymers, for instance poly(ethylene oxide), triblock copolymers of the poly(alkylene oxide) type, such as those of the ethylenoxide/propylenoxide/ethylenoxide type, or diblock copolymers of the poly(alkylene oxide) type, such as those of the ethylenoxide/butylenoxide-type, the size of the templating agent can be roughly adjusted by selecting polymers having a suitable molecular weight. The polymers of the (alkylene oxide) type mentioned above have preferably a molecular weight (number average molecular weight) of 1,100 - 15,000, more preferably 2,900 - 8,400, and most preferably 4,200 - 6,000. The number average molecular weight can be determined by GPC (gel permeation chromatography), using polystyrol as standard.

Other usable non-ionic templating agents are surfactant-based agents, such as primary amines, e.g. those of the formula CₙH₂ₙ₊₁NH₂, wherein n is an integer of 12 to 18 and CₙH₂ₙ₊₁ represents a branched or, preferably, a linear alkyl group, or alcohols, such as primary alcohols, e.g. those of the formula CₙH₂ₙ₊₁OH, wherein n is an integer of 12 to 18 and CₙH₂ₙ₊₁ represents a branched or, preferably, a linear alkyl group.

The relative proportions of the templating agent and the metal precursors in the mixture provided in step (i) of the method of the present invention is not specifically limited, but preferably in the range of 0.001 - 0.03, preferably 0.01 to 0.05, expressed as [molar amount of templating agent/molar amount of metal precursors], . Specifically, the concentration of the metal precursors is preferably in the range from 0.3 - 1.3 mol/1, more preferably 0.5 - 0.8 mol/1, and most preferably 0.6 - 0.7 mol/1, and the concentration of the templating agent is preferably in the range from 0.001 - 0.1 mol/1, more preferably 0.002 to 0.02 mol/1, and most preferably 0.005 to 0.01 mol/1.

### STEP (ii)

The solution or slurry obtained in step (i) of the method of the present invention is then reacted in step (ii), preferably at a temperature of more than 100 °C, to form a mixed metal oxide network.

Whilst the exact temperature and pressure conditions can be freely selected depending on the type of metal precursor, solvent and templating agent used, the reaction is generally and preferably performed at a temperature in the range of 100 - 200 °C, more preferably at a temperature in the range of 110 -190 °C, even more preferably at a temperature in the range of 120 - 180 °C.

When a polymer (such as Pluronic 123) is used as templating agent, the reaction is preferably conducted at a temperature in the range of 110 - 150 °C, more preferably in the range of 120 - 140 °C, and most preferably at 130 °C.

Preferably, the reaction is performed in a pressure-resistant reaction vessel, such as an autoclave. This allows performing the reaction at higher temperatures as compared to the boiling point of the solvent used under normal pressure. For instance, the use of a pressure-resistant reaction vessel allows the use of aqueous solutions at temperatures exceeding 100 °C.

In the most preferred embodiment of the reaction of step (ii) of the method of the present invention, water is used as solvent, the reaction is conducted in an autoclave, and the reaction temperature is 130 °C. Such a reaction is also called hydrothermal synthesis.

Preferably, the reaction of step (ii) of the method of the present invention is carried out in a (optionally pressure-resistant) reaction vessel in which the mixture obtained in step (i) can be mixed and/or stirred. This can be achieved by using, for example, an autoclave provided with a stirring means (e.g. by placing a magnetic stir bar in the autoclave and a magnetic induction stirrer under the autoclave) or a rotating autoclave.

If the reaction of step (ii) of the method of the present invention is carried out in a pressure-resistant vessel, it is preferable to remove unnecessary gases (in particular oxygen) from the mixture obtained in step (i) prior to sealing the pressure-resistant reaction vessel. This can for example be achieved by purging the mixture several times with nitrogen, or by sonification.

The duration of step (ii) is not limited, as long as the reaction time is sufficient for the formation of a mesoporous catalyst. That is, the time has to be sufficient for the metal precursors to form a network around the templating agent. Of course, the necessary reaction time depends on the kind of metal precursor, its amount, the templating agent used, whether or not promoting and/or inhibiting additives are present, the reaction temperature and the pressure applied. Generally, the reaction of step (ii) is preferably performed for 2 hours to 6 days, more preferably 1 day to 5 days, and most preferably 3 - 4 days.

The material obtained from step (ii) is a metal oxide network consisting essentially of the metal oxides, derived from the respective metal precursors, and the templating agent inside the pores. The material can be filtered or centrifuged/decanted, washed and/or dried or subjected to another conventional treatment before used in step (iii) of the method of the present invention.

Preferably, the product obtained from step (ii) is washed with water after its removal from the reaction vessel. Also preferably, the drying temperature does not exceed 100 °C.

### STEP (iii)

In step (iii) of the method of the present invention, the templating agent is removed from the material produced in step (ii).

Step (iii) of the method of the present invention comprises a step in which the material obtained in step (ii) is calcined at temperature in the range of 300 - 700 °C, preferably between 400 - 680 °C, even more preferably 560 - 660 °C (e.g. 600 - 620 °C), in a substantially oxygen-free atmosphere. The term "substantially oxygen-free atmosphere", as it is used here, refers to an atmosphere with a concentration of oxygen of 100 ppm or less, preferably 10 ppm or less, and most preferably 5 ppm or less. For example, it is in a preferred embodiment possible to perform step (iii) in an argon atmosphere containing 2 ppm of oxygen, commercially available as Argon 5.0. If the concentration of oxygen is higher than 100 ppm, oxidation of the material synthesized in step (ii) may occur, potentially leading to metal species having an oxidation state too high for catalytic applications. Preferably, the calcination is performed under inert gas atmosphere, that is, under a nitrogen, helium or argon atmosphere having an oxygen concentration of less than 100 ppm, preferably 10 ppm or less, and most preferably 5 ppm or less. In view of its inertness it is most preferred to conduct the calcination in an argon or helium atmosphere.

The duration of the calcination treatment is not particularly limited. Generally, the duration should be such that the templating agent is completely removed from the material. Naturally, the exact temperature and the duration of the calcination depend on the kind of material and the templating agent used, but generally a calcination treatment of 1 - 10 hours is considered to be sufficient.

It is possible to perform the calcination by placing the material obtained in step (ii) in an oven set to the desired temperature (i.e. an oven having a temperature within the above-described ranges). However, if the heating of the material obtained in step (ii) is too rapid, the templating agent may decompose rather than evaporate from the pores of the material. This may lead to the deposition of decomposition products, which is undesirable in view of the catalytic performance of the resulting material. The calcination is therefore preferably performed by placing the material obtained in step (ii) in an oven at a temperature from room temperature to 100 °C, removing the oxygen from the oven by evacuating or by introducing a flow of inert gas (such as nitrogen, helium and/or argon) and raising the temperature of the oven at a rate of up to 40 °C/min, preferably 5 to 30 °C/min, and most preferably 10 - 20 °/min, until the desired end temperature of the calcination is reached. In order to facilitate the removal of the template from the material, it is preferred to pass a flow of inert gas (e.g. nitrogen, helium and/or argon) at a flow rate of up to 200 ml/min, preferably 80 - 120 ml/min, over the material to be calcined, so that the (at the calcination temperature gassy) templating agent is removed and cannot be re-deposited on the material when the temperature is lowered after the end of the calcination.

It is optionally also possible to perform a preheating treatment at temperatures between 100 - 350 °C, preferably 150 - 300 °C, more preferably 175 - 250 °C in an atmosphere that is not substantially oxygen free, i.e. in an atmosphere having an oxygen concentration greater than 100 ppm. However, the conditions are preferably adjusted such that no MoO₃ is formed in the case of Mo-containing catalysts, as the presence of MoO₃ may cause over-oxidation of the organic substrate to CO/CO₂ when the final material is used as catalyst. This preheating treatment can be combined with the calcination treatment explained above, for example by raising the temperature up to 250 °C in an oxygen-containing atmosphere, then switching the gas flow to a substantially oxygen-free atmosphere or evacuating the oven accordingly, so that the oxygen concentration is below 100 ppm, and then performing the calcination as described above.

In a preferred embodiment of the process of the present application, the step (iii) for removing the templating agent from the material synthesized in step (ii) comprises an extraction step in order to remove the templating agent at least partially from the material obtained in step (ii). Said extraction step is performed prior to the calcination described above.

The technique used for extracting the templating agent in said extraction step is not particularly limited, as long as it is capable of removing at least some of the templating agent from the material prepared in step (ii). As suitable extraction methods there can be mentioned immersing the material obtained in step (ii) in a solvent, or Soxhlet extraction with such a solvent. Usable solvents in this respect can be selected from those known to the skilled person, and specifically oxygen- or nitrogen-containing solvents are usable. Examples of the oxygen-containing solvents include ketones, such as acetone, cyclohexanone or methyl ethyl ketone (MEK) etc., alcohols, such as methanol, ethanol, i-propanol, tert.-butanol etc., ethers, such as dimethylether, diethylether, furane, or tetrahydrofurane (THF), and lactones, such as ε-caprolactam. Examples of the nitrogen-containing solvent include amines, such as ethylamine, N,N-dimethylamine, triethylamine, amides, such as acetamide, heterocyclic nitrogen-containing solvents, such as pyridine, primidine, pyrazol, etc. Also other polar solvents can be used, such as CS₂.

If the templating agent is a polymer (in particular a block copolymer of the poly(alkylene oxide) type, such as those of the Pluronic series), a preferred extraction solvent is acetone. However, the extraction solvent used is not particularly limited, as long as it can dissolve the templating agent at least to some degree (i.e. in an amount of 1g/1l of solvent).

The degree to which the templating agent is removed by extraction prior to the calcination is not particularly limited, but it is preferably that at least 10%, more preferably at least 30%, and still more preferably at least 50% by weight of the templating agent is removed by extraction prior to the calcination. The amount to which the templating agent is removed prior to extraction can be calculated on the basis of the weight difference of the material before and after the extraction treatment, taking the weight loss of a sample of 0.1 g between room temperature and 625 °C observed by thermogravimetric analysis (TG) at a heating rate of 5 °C/min at a gas flow of 100 ml/min of 21% oxygen in argon as a basis. A usable apparatus for performing the measurement is STA 449 C Jupiter apparatus (Netzsch).

In a preferred embodiment of the present invention, the above-described step (iii) is performed in a rotating oven.

### B) Catalyst of the invention

The catalyst of the invention is obtainable by the method according to the invention described above.

The material obtained from step (iii) is a mixed metal oxide material having a mesoporous structure. It is preferably substantially free of crystalline domains having long range atomic order (i.e. does not show well-defined diffraction peaks, as defined below)), and it has preferably a specific surface area (BET surface area) of at least 20 m²/g, more preferably at least 50 m²/g, even more preferably at least 70 m²/g, and most preferably at least 100 m²/g. The upper limit of the BET surface area of the catalyst of the invention in not specifically limited, but it is typically less than 1,000 m²/g, e.g. less than 800 m²/g or less than 500 m²/g. The method for measuring the BET surface area is defined below.

Measurement of nitrogen adsorption at -196°C can be performed using, e.g., the Autosorb-1 instrument (Quantachrome). The Brunauer-Emmett-Teller (BET) method is used to determine the surface area of solid materials. The instrument measures the amount of nitrogen adsorbed onto the catalyst surface at some equilibrium vapour pressure by the static volumetric method. The surface area is calculated from the amount of adsorbed nitrogen forming a monolayer assuming that the molecular cross-sectional area for nitrogen is 16.2 Å². Prior to the adsorption, the samples have to be treated in vacuum several hours at temperatures between 80-300°C to remove molecules adsorbed from the environment. The catalyst of the invention is typically evacuated for 4 h at 80°C. Afterwards, known quantities of N₂ are admitted into a sample cell containing the catalyst maintained at -196°C. The BET analysis requires a linear plot of the amount of the adsorbed nitrogen at a relative pressure p/po (p is the equilibrium vapour pressure measured, po is the equilibrium vapour pressure of liquid nitrogen), which is restricted to a limited region of the adsorption isotherm, usually in the p/po range 0.05 to 0.3. The BET surface area of the catalyst of the invention is determined by measuring 5 points in this linear relative pressure range. The amount of sample is around 0.1 g.

Moreover, the carbon content of the material, as determined by combined thermogravimetric and differential scanning calometric analysis with gas phase analysis by mass spectrometry (TG-DSC-MS) of the material obtained from step (iii) using a STA 449 C Jupiter apparatus (Netzsch) and a thermostar quadrupole mass spectrometer (Pfeiffer Vacuum) is preferably less than 10%, more preferably less than 5 %, and most preferably less than 2 % by weight.

The catalysts of the present invention are preferably prepared by a preparation method involving a calcination step in a substantially oxygen-free atmosphere, as described above for the method of the present invention, in contrast to the preparation method described by Florea et al in Catalysis Today, 2006, 112, 139. Due to this process step the materials can maintain their mesoporous structure, which is favorable for their application as catalysts.

In one preferred embodiment the catalysts prepared in accordance with the method of the invention are preferably free of crystalline domains having long range atomic order (i.e. show no well-defined diffraction peaks, as defined below), in particular domains of MoO₃ in the case that the catalysts comprise Mo. This can also contribute to the catalytic performance of these materials. In this context it should be noted that crystalline MoO₃ may cause over-oxidation of the substrate (i.e. combustion to CO and CO₂), and consequently the presence of such crystalline domains is undesirable.

As discussed above, the materials obtained in accordance with the method of the present application have identical or highly similar electronic properties as the respective bulk materials. However, if for the intended use of the obtained material electronic properties (oxidation states) different from those of the materials obtained from step (iii) are required, the material can optionally be subjected to a reducing or oxidative post treatment. The methods applicable therefore are known to the person skilled in the art, and any method can be used, as long as the benefits of the present invention are not sacrificed.

The catalysts of the invention preferably comprise at least one transition metal, which is preferably molybdenum. More preferably, the catalyst comprises at least two transition metals selected from Mo, V and Nb. Even more preferably, the catalyst comprises all of these three elements, and it is still more preferred that the catalyst comprises these three elements (V, Nb and Mo), and additionally Te, most preferably forming a composition according to the formula (I) shown below:

movₐTe_{b}Nb_{c}Z_{d}Oₓ (I)

wherein a = 0.0-0.50, preferably 0.15-0.40, b = 0.0-0.45, preferably 0.10-0.40, c = 0 - 0.5, preferably 0.07 - 0.25, d ≤ 0.05, preferably 0.03 or less, and x is a number depending on the relative amount and valence of the elements different from Oxygen in formula (I), and Z is at least one element selected from Ru, Mn, Sc, Ti, Cr, Fe, Co, Ni, Cu, Zn, Ga, Y, Zr, Rh, Pd, In, Sb, Ce, Pr, Nd, Te, Sm, Tb, Ta, W, Re, Ir, Pt, Au, Pb, and Bi, provided that at least two, preferably at least three different metal species are contained in the catalyst composition, that is, one, preferably at least two of a, b, c, and d are not zero. "Average" composition means the composition as can be determined with techniques such as XRF suitable for analyzing the bulk elemental composition.

Preferably, in formula (I) a = 0,15-0,40, b = 0,10-0,40, c = 0,07-0,25, and d = 0,03 or less, more preferably a = 0,15-0,30, b = 0,15-0,25, c = 0,10-0,23 and d = 0,01 or less.

According to one preferred embodiment of the present invention d is 0 in formula (I).

If in the compound of the formula (I) the at least one optional element Z is present (i.e. d > 0), it is preferably at least one element selected from Ru, Mn, Cr, Fe, Co, Ni, Zr, Rh, Pd, In, Sb, Ce, Ta, W, Pt, and Bi. More preferred are compounds of formula (I), wherein Z, if present, is at least one element selected from Cr and Ni. Another preferred embodiment relates to the use of Ru, Cu, Rh, Re and/or Mn as Z element, Ru, Mn and Cu, in particular Ru and Mn being particularly preferred. If element Z is present, the lower limit of d is preferably 0.0005, in particular 0.001.

More preferred catalysts have the stoichiometric composition Mo₁V_{0.19-0.25}Te_{0.19-0.23}Nb_{0.12-0.20}Oₓ, wherein x is defined as in formula (I) above.

The compositions of the catalysts of the invention mentioned above, such as those described by formula (I) above, relate to the composition of the bulk material (i.e. the average composition over the whole of the material), as determined for example by XRF. However, the composition of the catalyst surface may differ from the bulk composition. In particular, in the case of MoVTeNb catalysts, sometimes a lower amount of certain elements, in particular V and/or Nb, is found when the catalyst surface is examined with a surface-specific analytic technique, such as XPS. Accordingly, the surface composition of the catalysts of the invention preferably fulfills the following formula (Ia) :

MoVₑTe_{f}Nb_{g}ZₕOx (Ia)

wherein e = 0.0-0.50, preferably 0.10-0.35, more preferably 0.10 - 0.25, f = 0-0.45, preferably 0.05-0.35, more preferably 0.10 - 0.25, g = 0 - 0.5, preferably 0.05 - 0.25, more preferably 0.07 - 0.21, and d < 0.05, preferably 0.03 or less, and x is a number depending on the relative amount and valence of the elements different from Oxygen in formula (I), and Z is at least one element selected from Ru, Mn, Sc, Ti, Cr, Fe, Co, Ni, Cu, Zn, Ga, Y, Zr, Rh, Pd, In, Sb, Ce, Pr, Nd, Te, Sm, Tb, Ta, W, Re, Ir, Pt, Au, Pb, and Bi, provided that at least two, preferably at least three different metal species are contained in the catalyst composition, that is, one, preferably at least two of a, b, c, and d are not zero. As mentioned above, the surface composition can be determined by XPS. For example, the surface composition can be determined by the following procedure:
In a typical experiment, the XPS measurement is performed using a modified LHS/SPECS EA200 MCD system and Mg K_{α} radiation. The samples are used as loose powders to avoid any damaging of the surface layer. The compositions are obtained after subtracting satellites and stepped background and and by integration using empirical cross sections of the raw data obtained in the pass energy mode (48 eV PE giving 0.9 eV FWHM of the Ag35/2 intensity). For the specifics of the XPS analysis, reference can be made to D. Briggs, M. P. Seah, Practical Surface Analysis, Wiley, 1990.

The catalysts of the invention are mesoporous materials. The term "mesoporous" as used in the present invention denotes a material having pores with a pore size in the range of 2 - 50 nm. In the invention, the size of the pores can be measured by a nitrogen adsorption technique as follows:
The catalyst (e.g. obtained after step (iii) of the method of the invention) can be analyzed for its pore size and pore size distribution according to the following method: Nitrogen adsorption-desorption isotherm is measured at 77 K using for example an Autosorb-1 instrument (Quantachrome). Previous to the adsorption, the sample is outgassed in vacuum at 353 K for 4 h. Calculation of the pore size distribution is performed using the desorption branch of the isotherm and the Barrett-Joyner-Halenda (BJH) method, as described in E.P. Barrett, L.G. Joyner, P.P. Halenda, J. Amer. Chem. Soc. 73 (1951) 373.. Full adsorption/desorption isotherms in the p/p₀ range 0.001 to 1 were recorded. The Quantachrome AUTOSORB software was used to calculate the pore size distribution based on the complete desorption branch of the isotherm and the Barret-Joyner-Halende (BJH) method. The sample size is around 0.1 g.

In the present invention, a material is preferably considered to be "mesoporous" if at least 50%, more preferably at least 70%, and most preferably at least 90% of the total pore volume (calculated according to the BJH method, using the nitrogen adsorption experiment described above) is present in pores having a diameter between 2 - 50 nm.

The nitrogen adsorption-desorption isotherm shows a hysteresis loop between 0.4 - 1.0 relative pressure (p/p₀) (Type IV isotherm, as described for example in K.S.W. Sing, D. H. Everett, R. A. W. Haul, L. Moscou, R. A. Pierotti, J. Rouquerol, T. Siemieniewska, Pure & Appl. Chem. 57 (1985) 603 - 619)).

Preferably, the BJH pore size distribution of the catalyst of the invention has a maximum peak in the range of 2 - 16 nm, more preferably 4 - 14 nm, and most preferably 5 - 12 nm. The BJH pore size distribution is obtained according to the nitrogen adsorption technique described above.

The mesoporosity of the catalyst can also be confirmed by Scanning Electron Microscopy (SEM). The porous nature is clearly visible with pore openings varying from 7 to 12 nm in diameter. The sample morphology can for example be studied using a Hitachi S-4800 FEG scanning electron microscope. The dry sample is crushed using a mortar and pestle. The crushed powder is dispersed in dry form on a carbon coated copper grid. Secondary electron (SE) images are acquired at an accelerating voltage of 2 kV. For acquisition of energy-dispersive X-ray (EDX) spectra, the acceleration voltage is increased to 10 kV.

The weight loss of the catalyst after template removal/calcination, when subjected to thermal analysis (TG) after being calcined as described in step (iii) of the method of the invention at a temperature of 600 C for 2 hours in a rotating oven with a heating rate of 15 °C/min, which is observed in a temperature range from room temperature to 625 °C, is preferably less than 15 wt.-%, more preferably less than 10 wt.-%.

As described above, the catalyst of the invention is a mesoporous material. In a preferred embodiment, such materials are preferably not crystalline, i.e. do not exhibit long-range atomic order. Consequently, the catalyst of this preferred embodiment is preferably amorphous, i.e. its XRD pattern is substantially free of well-defined diffraction peaks. Herein, and as used above, the term "well-defined diffraction peak" is to be understood as relating to a diffraction signal having an FWHM (full width at half maximum), i.e. the width of the peak at 50% of its height above the baseline, of at most 3° in 2 theta.

However, whilst preferably no crystalline domains having long range atomic order are present, the pore system of the catalyst of the invention may exhibit long-range order, which is independent of whether or not the wall material as such is amorphous or crystalline. Such a long-range ordering of the pores may lead to diffraction peaks, but at lower diffractions angles as compared to diffraction peaks resulting from crystallinity of the wall material. Accordingly, the catalyst may exhibit diffraction peaks at bigger d-spacings (i.e. at lower angles), that is, d-spacings of more than 20 Angstrom (2 nm). Accordingly, it is preferred that the catalyst of the invention does not exhibit diffraction peaks at d-spacings of less than 2 nm, whilst it may exhibit diffraction peaks at d-spacings of 2 nm or more.

The XRD pattern of the catalyst can be obtained by using a STOE STADI-P transmission diffractometer equipped with a focusing primary Ge (111) monochromator and a position-sensitive detector (PSD).

Preferably, the catalyst of the invention does not exhibit diffraction peaks at angles greater than 5° in 2 theta using Cu Kα radiation. Most preferably, the X-ray diffraction pattern using the above instrument is free of peaks attributable to crystalline Mo^{VI} species, in particular MoO₃.

### EXAMPLES

In the following, the present invention will be described with reference to Examples. However, these are not intended to limit the scope of the invention in any way.

### Test methods and analysis

### Physisorption of nitrogen

Measurement of nitrogen adsorption at -196°C was performed using the Autosorb-1 instrument (Quantachrome). The Brunauer-Emmett-Teller (BET) method was used to determine the surface area of solid materials. The instrument measures the amount of nitrogen adsorbed onto the catalyst surface at some equilibrium vapour pressure by the static volumetric method. The surface area is calculated from the amount of adsorbed nitrogen forming a monolayer assuming that the molecular cross-sectional area for nitrogen is 16.2 Å². Previous to the adsorption, the samples have to be treated in vacuum several hours at temperatures between 80-300°C to remove molecules adsorbed from the environment. The catalyst of the invention was evacuated for 4 h at 353 K. Afterwards, known quantities of N₂ are admitted into a sample cell containing the catalyst maintained at -196°C. The BET analysis requires a linear plot of the amount of the adsorbed nitrogen at a relative pressure p/po (p is the equilibrium vapour pressure measured, po is the equilibrium vapour pressure of liquid nitrogen), which is restricted to a limited region of the adsorption isotherm, usually in the p/po range 0.05 to 0.3. Full adsorption/desorption isotherms in the p/p₀ range 0.001 to 1 were recorded. The Quantachrome AUTOSORB software was used to determine the BET surface area of the catalyst of the invention taking into account 5 data points in the linear relative pressure range of the adsorption isotherm (p/p₀ pressure range 0.05 to 0.3) The same software was applied to calculate the pore size distribution based on the complete dsorption branch of the isotherm (40 data points in the pressure range 1 to 0.001) and the Barrett-Joyner-Halenda (BJH) method. The amount of sample is around 0.1 g.

### Photoelectron spectroscopy (XPS)

XPS measurements were performed using a modified LHS/SPECS EA200 MCD system and Mg Kα radiation. Samples were used as loose powders to avoid any damaging of the surface layer. Compositions were obtained after subtracting satellites and stepped background and integration using empirical cross sections (D. Briggs, M. P. Seah, Practical Surface Analysis, Wiley, 1990.) of the raw data obtained in the pass energy mode (48 eV PE giving 0.9 eV FWHM of the Ag35/2-intensity).

### X-Ray Diffraction

X-ray diffraction measurements were performed with a STOE STADI-P transmission diffractometer equipped with a focussing primary Ge (1 1 1) monochromator and a position sensitive detector (PSD), using Cu Kα₁ radiation. Small angle XRD was performed with the same setup described above, by replacing the PSD with a scintillation counter and removing the beam stop. The detector slit was set to 0.1 mm, while the standard collimator was replaced with a narrow collimator designed for capillary measurements.

### SEM and EDX

The sample morphology was studied using a Hitachi S-4800 FEG scanning electron microscope (SEM). For transmission electron microscopy (TEM) studies, a Philips CM 200 microscope with a field emission gun operating at 200kV was used. The dry sample was crushed using a mortar and pestle. The crushed powder was dispersed in dry form on a carbon coated copper grid. Secondary electron (SE) images were acquired at an accelerating voltage of 2 kV. For acquisition of energy-dispersive X-ray (EDX) spectra, the acceleration voltage was increased to 10 kV. The same grid was used for SEM and TEM studies. In order to calculate the bulk composition of a given compound via EDX, several measurements have to combined and averaged due to the area covered by EDX being relatively small. A meaningful value can be obtained by averaging the values obtained from 50 EDX measurements.

### Thermal Analysis

Combined thermogravimetric and differential scanning calorimetric analysis with gas phase analysis by mass spectrometry (TG-DSC-MS) was performed using a STA 449 C Jupiter apparatus (Netzsch) and a Thermostar quadrupole mass spectrometer (Pfeiffer Vacuum). About 20 mg of sample were heated to 873 K at 5 K/min in 100 ml/min of 21% oxygen in argon (Westfalen, 5.0).

### Oxidation Test

Partial oxidation of propane was carried out in a reactor for parallel catalytic testing with twelve fixed bed quartz tubular reactors (i.d., 4 mm; length, 225 mm), working at atmospheric pressure. The catalyst was pressed by a hydraulic press (8 ton of force on a round surface of approx. 3cm diameter), crushed and sieved manually to particle sizes between 0.24 to 0.45 mm. The feed flow rate was fixed at a gas hourly space velocity (GHSV) of 4800 h⁻¹ (at STP) with a catalytic bed volume of 0.125 ml that corresponds to 1 cm in bed height The feed composition was 2.8 vol-% propane, 6.3 vol-% oxygen, 50.9 vol-% nitrogen and 40 vol-% steam. The reaction was carried out at 673 K. The products were analyzed by two on-line gas chromatograph systems. The first GC was configured with molecular sieve and Porapak columns coupled with a thermal conductivity detector for the analysis of inorganic gases and hydrocarbons (C1-C3). The second GC was configured with a capillary column (HP-FFAP) coupled with a flame ionization detector for the analysis of oxygenated products.

### PREPARATION EXAMPLES

### Inventive Example:

Aqueous metal salt solutions with the metals in a stochiometry Mo₁V_{0.25}Te_{0.23}Nb_{0.124} were mixed with the triblock copolymer poly-(ethylene glycol)-block-poly-(propylene glycol)-block-poly-(ethylene glycol), Mₙ ca. 5,800 (Aldrich) in a molar ratio polymer/metal = 0.011. 7.096 g ammonium heptamolybdate (NH₄)₆Mo₇O₂₄ x 4 H₂O (Merck), 2.542 g vanadyl sulphate VOSO₄ x H2O (Fluka) and 2.123 g hexaoxotelluric acid Te(OH)₆ (Aldrich) were dissolved in 100 ml bidestilled water at 353 K and subsequently cooled to 333 K. 4.18 g of the polymer in 30 ml of water was added to the solution under stirring. The resultant gel was stirred for 10 min. Afterwards, a solution of 2.168 g ammonium niobium oxalate (NH₄)₂Nb₂(C₂O₄)₅ (Aldrich) in 30 ml water dissolved at 313 K was added. The gel was stirred for another 15 minutes at 333 K and subsequently transferred to a teflon-lined autoclave. After four days at 403 K, the purple colored product was filtered, washed twice with 100 ml water and dried overnight in air at 353 K. The obtained material was subjected to Soxhlet-extraction for 24 hours. Soxhlet-extraction was performed with 3.5 g of the as-synthesized material applying acetone as solvent. The extraction with acetone resulted in the removal of about 30% of the polymer after 24 hours. Finally, the extracted material was treated at 873 K (heating rate 15 K min-1) in argon 5.0 for 2 h in a rotating oven.

### Reference Example:

A reference material was prepared in accordance with the inventive example described above, except that no polymer (templating agent) was used in the synthesis.

### Evaluation:

The materials obtained from the inventive example and the reference example had the bulk composition (measured by EDX, as described above) and surface composition (measured by XPS, as described above) shown in the following table:

| Sample | Measurement technique | Composition |
|---|---|---|
| Inventive Example | XPS (surface composition) | MOV_{0.13}Te_{0.20}Nb_{0.12}O_{3.68} |
| | EDX (bulk composition) | MoV_{0.19}Te_{0.19}Nb_{0.20}Oₓ |
| Reference Example | XPS (surface composition) | MoV_{0.11}Te_{0.14}Nb_{0.18}O_{3.48} |
| | EDX (bulk composition) | MoV_{0.11}Te_{0.18}Nb_{0.23}Oₓ |

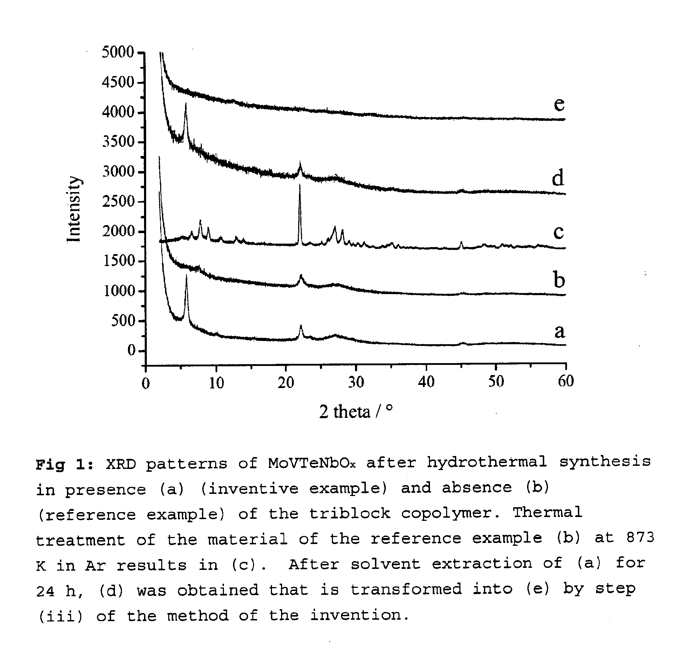

In the inventive example, after hydrothermal synthesis at 403 K for 96 h in the presence of the polymer, a black-purple colored powder was isolated showing similarities in appearance with the reference material prepared under identical conditions in absence of the polymer. The lack of small angle x-ray diffraction lines (not shown) indicates the absence of long-range ordered pore structure in the MoVTeNb oxide. Three peaks at 2θ angles of 5.8, 22.3 and 27.1 are observed in the XRD patterns (Fig. 1a). The latter two features are due to nano-crystalline MoVTeNb oxide precursors. Similar peaks are observed for the reference material synthesized in absence of the polymer (Fig. 1b). Crystallization of this reference material in inert atmosphere resulted in a phase mixture composed of 89% M1 and 11% M2 (Fig. 1c). Phase formation involves a drop in specific surface area from initially 216 m²g⁻¹ for the hydrothermally synthesized precursor to 1 m²g⁻¹ for the crystalline product. The peak at 5.8 in the diffractogramm of the polymer-containing material (Fig. 1a) according to the invention originates from the polymer, which has been partially removed by Soxhlet extraction with acetone for 24 h (Fig. 1d).Subsequent high temperature treatment in Ar resulted in a mixture of binary and mixed oxides with low specific surface area.

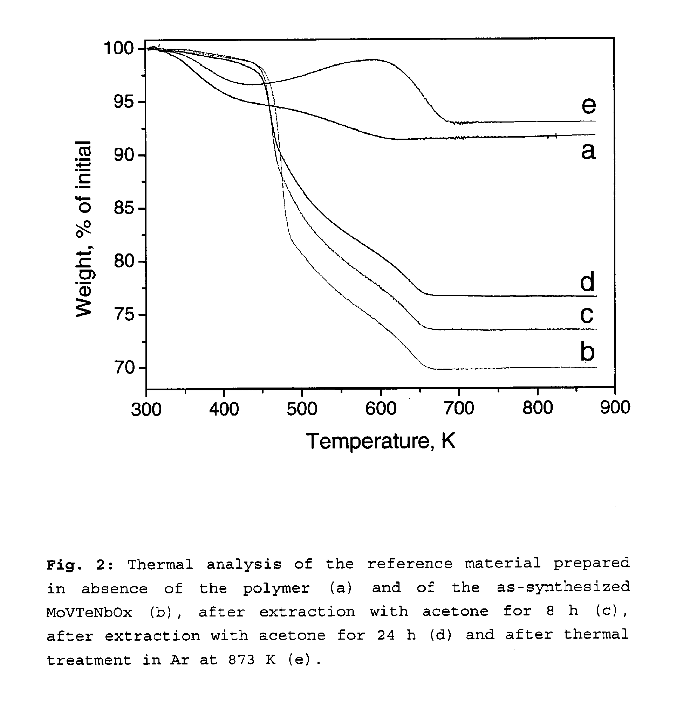

Thermal analysis of the reference material prepared in absence of the polymer shows an initial 5.5 % weight loss of H₂O followed by the decomposition of residual counter ions up to 618 K and then a weight gain of 0.3 % due to re-oxidation of the mixed oxide (Fig. 2a). The as-synthesized polymer containing material according to the invention shows two major weight losses when increasing the temperature by 5 K/min in air (Fig. 2b). A rapid decomposition / oxidation of the polymer and oxidation of ammonia, responsible for ca. 18 % weight loss, occurs between 440 and 483 K. The sample then loses additional 12 % weight between 486 and 663 K with the gas phase products of H₂O CO₂ and CO. This relatively constant rate of loss in weight, and the production of CO are indicative of a diffusion limited oxidation of the polymer from the pore system. The last 3 % weight loss between 610 and 663 K is more rapid and has a lower concentration of H₂O indicating the oxidation of carbonaceous deposits form the pore system and perhaps the decomposition of residual oxalate counter ions. The polymer containing material after extraction is mainly different in that it loses less weight during the initial rapid polymer decomposition at temperatures between 440 and 483 K, suggesting that the extraction removes polymer from larger pores and surfaces, but not from the smaller pores (Fig. 2c and 2d). Thermal analysis of the pyrolysed sample shows an initial loss of water followed by a re-oxidation weight gain of more than 2 % and above 590 K oxidation of carbonaceous deposits resulting in a weight loss of 6 % (Fig 2e).

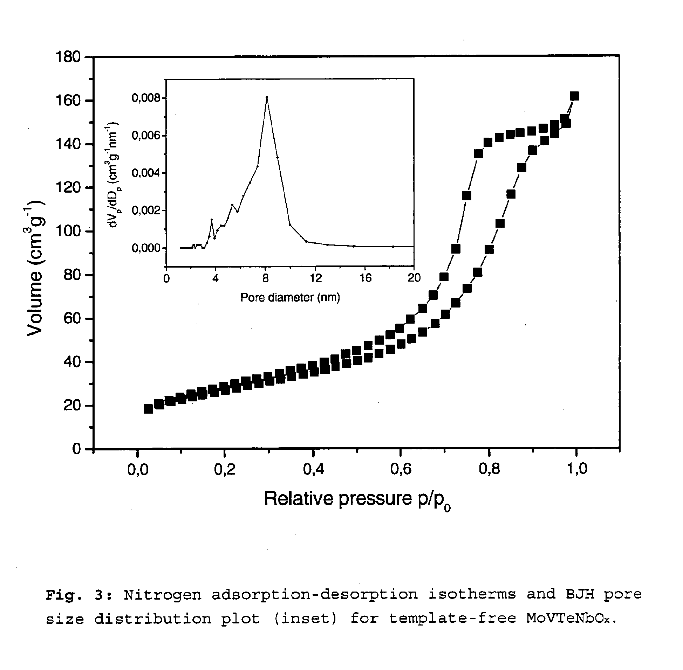

Nitrogen adsorption-desorption analysis of thermally treated MoVTeNbOx according to the invention (Fig. 3) showed a type IV isotherm with a sharp inflection at p/po = 0.7 and a broad hysteresis loop, characteristic of capillary condensation in the channels of a mesoporous material. The calculation of the pore size distribution was performed using the desorption branch of the nitrogen adsorption isotherm and the Barrett-Joyner-Halenda (BJH) formula (Fig 3, inset). Accordingly, a broad pore size distribution in the range of 6-12 nm with the maximum around 8 nm has been revealed. The surface area of this novel mesoporous MoVTeNb oxide of the invention is 96.2 m²g⁻¹, much higher than that of standard MoVTeNbOx catalysts.

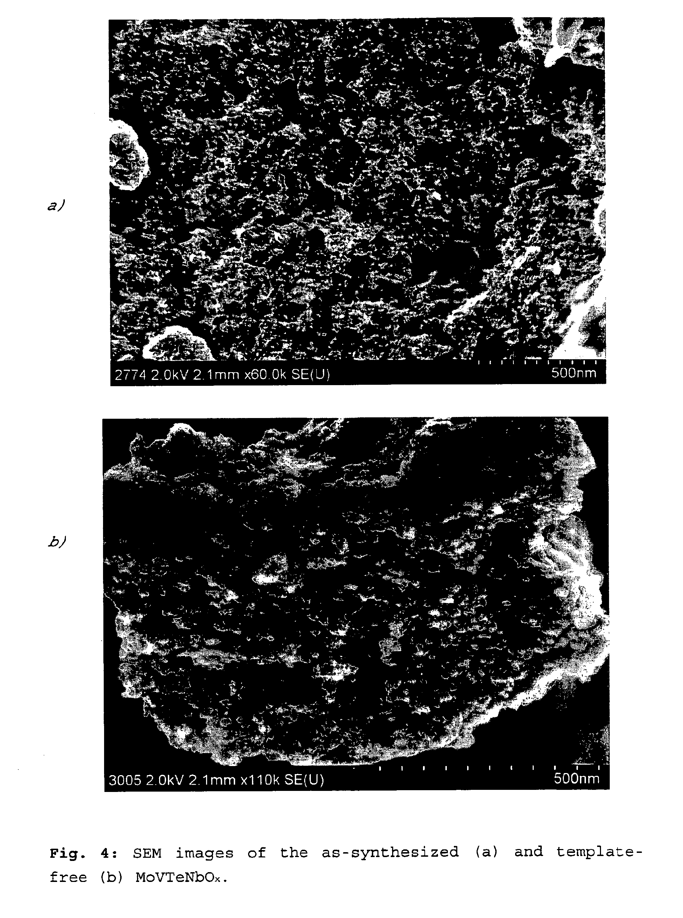

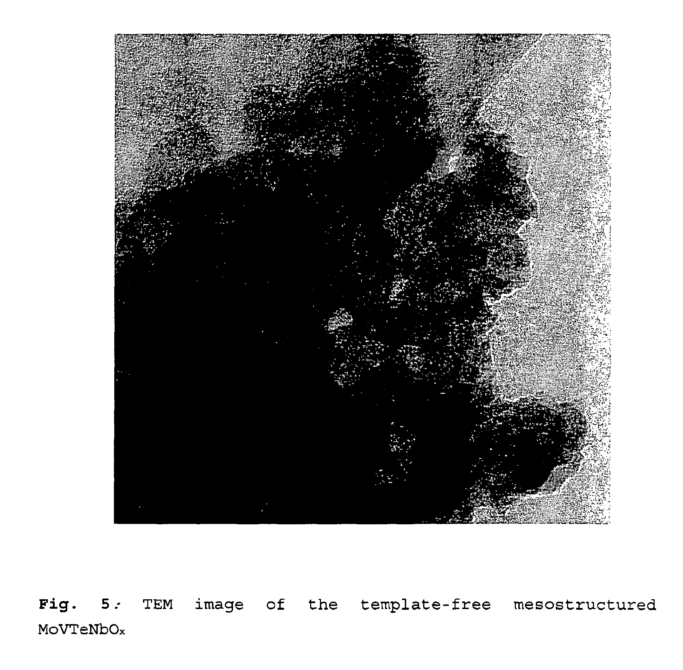

The SEM image of the hydrothermally synthesized MoVTeNbOx (Fig. 4a) shows similarities to a homogeneous sponge-type material. After removal of the polymer, the porous nature is clearly visible (Fig. 4b) with pore openings varying from 7 to 12 nm in diameter. In agreement with XRD, the TEM images confirm the irregular pore structure revealing mesopores progressing throughout the sample in a disordered fashion (Fig. 5).

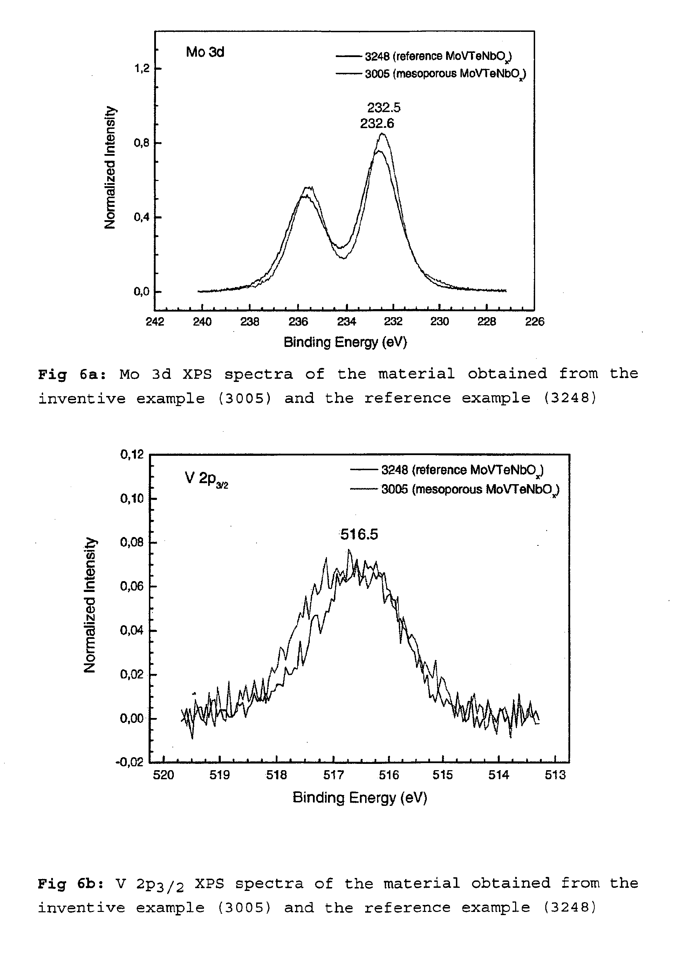

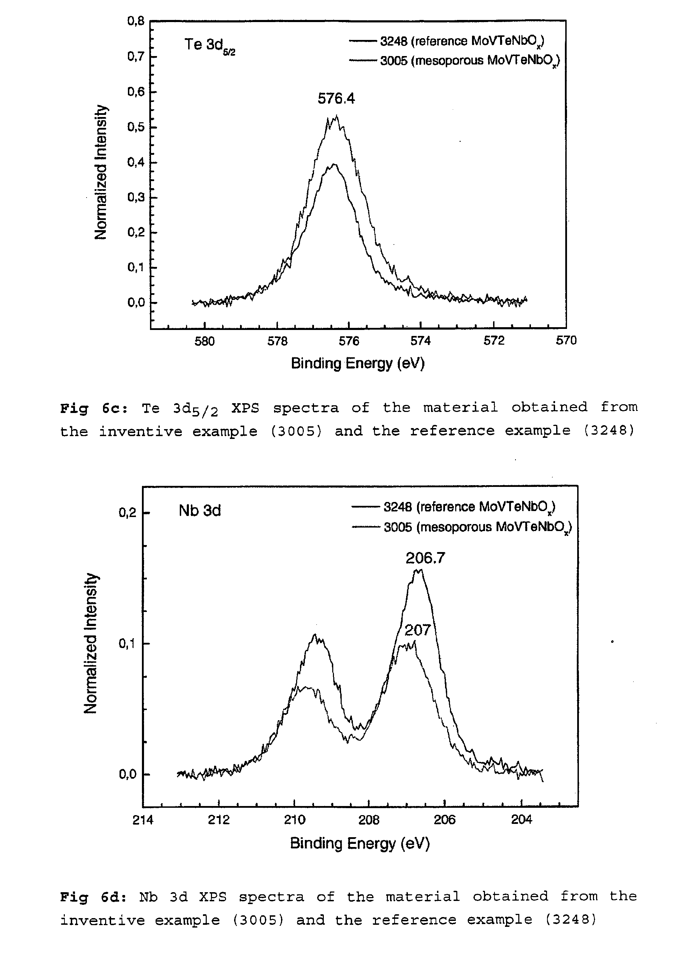

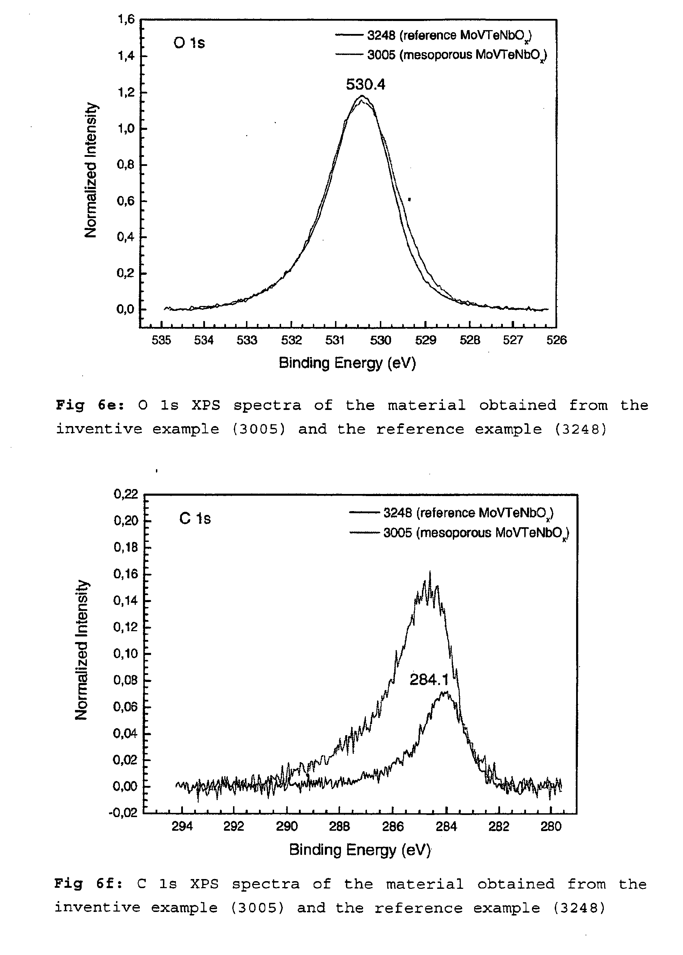

As can be seen from the XPS spectra in figures 6a - 6e, the catalyst of the example has similar electronic properties as compared to the bulk (reference) material.

### Partial oxidation of propane

In catalytic tests the mesoporous material shows stable activity in the selective oxidation of propane to acrylic acid. At 673 K a conversion of 8 % propane and a selectivity to acrylic acid of 77 % were measured.

## Claims

1. A method for the preparation of a mesoporous mixed metal oxide catalyst, comprising:
(i) a step of providing metal precursors and a non-ionic templating agent in a solution or slurry,
(ii) a step of reacting the mixture obtained in (i), and
(iii) a step of removing the template from the material obtained from (ii), comprising a calcination step in a substantially oxygen-free atmosphere at a temperature between 300 to 700°C.

2. The method according claim 1, wherein step (ii) is performed in a pressure-resistant vessel

3. The method according to any of claims 1 or 2, wherein step (ii) is performed at a temperature between 100°C and 200°C.

4. The method according to anyone of claims 1 to 3 above, wherein the template is at least partially removed by solvent extraction from the material obtained from step (ii) prior to the calcination treatment in step (iii).

5. The method according to any one of claims 1 - 4, wherein the metal precursors provided in step (i) comprise at least one transition metal.

6. The method according to claim 5, wherein the transition metal is molybdenum.

7. The method according to claim 6, wherein the metal precursors comprise at least two transition metals selected from Mo, V and Nb.

8. The method according to claim 7, wherein the metal precursors comprise Mo, V and Nb

9. The method according to claim 7 or 8, wherein the metal precursors additionally contain Te.

10. The method according to claim 9, wherein the obtained catalyst forms a composition according to the formula (I) shown below:
MoVₐTe_{b}Nb_{c}Z_{d}Oₓ (I)
wherein a = 0.0-0.50, preferably 0.15-0.40, b = 0-0.45, preferably 0.10-0.40, c = 0 - 0.5, preferably 0.07 - 0.25, d ≤ 0.05 and x is a number depending on the relative amount and valence of the elements different from Oxygen in formula (I), and Z is at least one element selected from Ru, Mn, Sc, Ti, Cr, Fe, Co, Ni, Cu, Zn, Ga, Y, Zr, Rh, Pd, In, Sb, Ce, Pr, Nd, Te, Sm, Tb, Ta, W, Re, Ir, Pt, Au, Pb, and Bi, provided that at least two, preferably at least three different metal species are contained in the catalyst composition, that is, one, preferably at least two of a, b, c, and d are not zero at the same time..

11. The method according to claim 10, wherein the obtained catalyst has a stoichiometric composition fulfilling the following formula:
Mo₁V_{0.15-0.3}Te_{0.15-0.25}Nb_{0.10-0.23}Oₓ wherein x is defined as in formula (I) in claim 10.

12. The method according to claim 11, wherein the obtained catalyst has a stoichiometric composition represented by
Mo₁V_{0.19-0.25}Te_{0.19-0.23}Nb_{0.12-0.20}Oₓ
wherein x is defined as in formula (I) in claim 10.

13. The method according to any one of claims 1 - 12, wherein the neutral templating agent is a polymer, a copolymer or a mixture thereof.

14. The method according to claim 13, wherein the copolymer contains oxygen.

15. The method according to claim 14, wherein the copolymer is of the poly(alkylene oxide) type.

16. A mesoporous catalyst, obtainable according to any of the methods of claims 1 -15.

17. The catalyst according to claim 16, wherein at least 50%, more preferably at least 70%, and most preferably at least 90% of the total pore volume is present in pores having a diameter between 2 - 50 nm.

18. The catalyst according to any of claims 16 or 17, wherein the catalyst does substantially not exhibit diffraction peaks at angles greater than 5° in 2 theta.

19. Use of a catalyst according to any of claims 16 - 18 for the partial oxidation of hydrocarbons.
